# EUROPEAN PATENT SPECIFICATION

(11) **EP 3 583 948 B1**
(45) Date of publication and mention of the grant of the patent: **06.04.2022**
(21) Application number: 18382452.3
(22) Date of filing: 20.06.2018
(51) Int. Cl.: A61K 39/12, C07K 14/005, A61K 39/295

(54) **VACCINES FOR THE PREVENTION OF RABBIT HAEMORRHAGIC DISEASE**
IMPFSTOFFE ZUR PRÄVENTION VON HÄMORRHAGISCHER KANINCHENKRANKHEIT
VACCINS POUR LA PRÉVENTION DE LA MALADIE HÉMORRAGIQUE DU LAPIN

(43) Date of publication of application: 25.12.2019
(73) Proprietor: FATRO S.p.A., I-40064 Ozzano Emilia (IT); Algenex S.L., 28223 Pozuelo de Alarcón (ES)
(72) Inventor: MORENO DALTON, Romy, 28223 Pozuelo de Alarcón (ES); ALVARADO FRADUA, Carmen, 28223 Pozuelo de Alarcón (ES); MARTÍNEZ ESCRIBANO, José Ángel, 28223 Pozuelo de Alarcón (ES)
(74) Representative: Hoffmann Eitle

(56) References cited:
- WO-A2-2017/046415
- PACHO SONSOLES ET AL: "Assessment of a Novel Vaccine Against Rabbit Hemorrhagic Disease Virus in Young Rabbits", VIRAL IMMUNOLOGY, vol. 29, no. 10, 1 December 2016 (2016-12-01), pages 583-585, XP009508543, ISSN: 0882-8245, DOI: 10.1089/VIM.2016.0093
- L. CAPUCCI ET AL: "Increased pathogenicity in rabbit haemorrhagic disease virus type 2 (RHDV2)", VETERINARY RECORD, vol. 180, no. 17, 24 March 2017 (2017-03-24), pages 426.2-426, XP055514119, GB ISSN: 0042-4900, DOI: 10.1136/vr.104132
- XUE WANG ET AL: "Atomic Model of Rabbit Hemorrhagic Disease Virus by Cryo-Electron Microscopy and Crystallography", PLOS PATHOGENS, vol. 9, no. 1, 17 January 2013 (2013-01-17), page e1003132, XP055514151, DOI: 10.1371/journal.ppat.1003132

### SEQUENCE LISTING

<110> FATRO SpA Alternative Gene Expression, SL
<120> Vaccines for the prevention of Rabbit haemorrhagic disease
<130> 201 855
<160> 8
<170> BiSSAP 1.3.6
<210> 1
   <211> 50
   <212> PRT
   <213> Artificial Sequence
<220>
   <223> NTA domain of the VP1 of RHDVa
<400> 1
   Met Glu Gly Lys Ala Arg Thr Ala Pro Gln Gly Glu Ala Ala Gly Thr
   1               5                   10                  15
   Ala Thr Thr Ala Ser Val Pro Gly Thr Thr Thr Asp Gly Met Asp Pro
               20                  25                  30
   Gly Val Val Ala Thr Thr Ser Val Val Thr Ala Glu Asn Ser Ser Ala
           35                  40                  45
   Ser Ile
       50
<210> 2
   <211> 529
   <212> PRT
   <213> Artificial Sequence
<220>
   <223> S and P domains of the VP1 of RHDVb
<400> 2
   Ala Thr Ala Gly Ile Gly Gly Pro Pro Gln Gln Val Asp Gln Gln Glu
   1               5                   10                  15
   Thr Trp Arg Thr Asn Phe Tyr Tyr Asn Asp Val Phe Thr Trp Ser Val
               20                  25                  30
   Ala Asp Ala Pro Gly Asn Ile Leu Tyr Thr Val Gln His Ser Pro Gln
           35                  40                  45
   Asn Asn Pro Phe Thr Ala Val Leu Ser Gln Met Tyr Ala Gly Trp Ala
       50                  55                  60
   Gly Gly Met Gln Phe Arg Phe Ile Val Ala Gly Ser Gly Val Phe Gly
   65                  70                  75                  80
   Gly Arg Leu Val Ala Ala Val Ile Pro Pro Gly Ile Glu Ile Gly Pro
                   85                  90                  95
   Gly Leu Glu Val Arg Gln Phe Pro His Val Val Ile Asp Ala Arg Ser
               100                 105                 110
   Leu Glu Pro Val Thr Ile Thr Met Pro Asp Leu Arg Pro Asn Met Tyr
           115                 120                 125
   His Pro Thr Gly Asn Pro Gly Leu Val Pro Thr Leu Val Leu Ser Val
       130                 135                 140
   Tyr Asn Asn Leu Ile Asn Pro Phe Gly Gly Ser Thr Ser Ala Ile Gln
   145                 150                 155                 160
   Val Thr Val Glu Thr Arg Pro Ser Glu Asp Phe Glu Phe Val Met Ile
                   165                 170                 175
   Arg Ala Pro Ser Ser Lys Thr Val Asp Ser Ile Ser Pro Ala Asp Leu
               180                 185                 190
   Leu Thr Thr Pro Val Leu Thr Gly Val Gly Thr Asp Asn Arg Trp Asn
           195                 200                 205
   Gly Glu Ile Val Gly Leu Gln Pro Val Pro Gly Gly Phe Ser Thr Cys
       210                 215                 220
   Asn Arg His Trp Asn Leu Asn Gly Ser Thr Phe Gly Trp Ser Ser Pro
   225                 230                 235                 240
   Arg Phe Ala Ala Ile Asp His Asp Arg Gly Asn Ala Ser Tyr Pro Gly
                   245                 250                 255
   Ser Ser Ser Ser Asn Val Leu Glu Leu Trp Tyr Ala Ser Ala Gly Ser
               260                 265                 270
   Ala Ala Asp Asn Pro Ile Ser Gln Ile Ala Pro Asp Gly Phe Pro Asp
           275                 280                 285
   Met Ser Phe Val Pro Phe Ser Gly Thr Thr Val Pro Thr Ala Gly Trp
       290                 295                 300
   Val Gly Phe Gly Gly Ile Trp Asn Ser Ser Asn Gly Ala Pro Phe Val
   305                 310                 315                 320
   Thr Thr Val Gln Ala Tyr Glu Leu Gly Phe Ala Thr Gly Ala Pro Ser
                   325                 330                 335
   Asn Pro Gln Pro Thr Thr Thr Thr Ser Gly Ala Gln Ile Val Ala Lys
               340                 345                 350
   Ser Ile Tyr Gly Val Ala Thr Gly Ile Asn Gln Ala Thr Ala Gly Leu
           355                 360                 365
   Phe Val Met Ala Ser Gly Val Ile Ser Thr Pro Asn Ser Ser Ala Ile
       370                 375                 380
   Thr Tyr Thr Pro Gln Pro Asn Arg Ile Val Asn Ala Pro Gly Thr Pro
   385                 390                 395                 400
   Ala Ala Ala Pro Ile Gly Lys Asn Thr Pro Ile Met Phe Ala Ser Val
                   405                 410                 415
   Val Arg Arg Thr Gly Asp Ile Asn Ala Glu Ala Gly Ser Thr Asn Gly
               420                 425                 430
   Thr Gln Tyr Gly Ala Gly Ser Gln Pro Leu Pro Val Thr Val Gly Leu
           435                 440                 445
   Ser Leu Asn Asn Tyr Ser Ser Ala Leu Met Pro Gly Gln Phe Phe Val
       450                 455                 460
   Trp Gln Leu Asn Phe Ala Ser Gly Phe Met Glu Leu Gly Leu Ser Val
   465                 470                 475                 480
   Asp Gly Tyr Phe Tyr Ala Gly Thr Gly Ala Ser Ala Thr Leu Ile Asp
                   485                 490                 495
   Leu Ser Glu Leu Val Asp Ile Arg Pro Val Gly Pro Arg Pro Ser Thr
               500                 505                 510
   Ser Thr Leu Val Tyr Asn Leu Gly Gly Thr Thr Asn Gly Phe Ser Tyr
           515                 520                 525
   Val
<210> 3
   <211> 579
   <212> PRT
   <213> Artificial Sequence
<220>
   <223> RHDVab chimera VP1 protein
<400> 3
   Met Glu Gly Lys Ala Arg Thr Ala Pro Gln Gly Glu Ala Ala Gly Thr
   1               5                   10                  15
   Ala Thr Thr Ala Ser Val Pro Gly Thr Thr Thr Asp Gly Met Asp Pro
               20                  25                  30
   Gly Val Val Ala Thr Thr Ser Val Val Thr Ala Glu Asn Ser Ser Ala
           35                  40                  45
   Ser Ile Ala Thr Ala Gly Ile Gly Gly Pro Pro Gln Gln Val Asp Gln
       50                  55                  60
   Gln Glu Thr Trp Arg Thr Asn Phe Tyr Tyr Asn Asp Val Phe Thr Trp
   65                  70                  75                  80
   Ser Val Ala Asp Ala Pro Gly Asn Ile Leu Tyr Thr Val Gln His Ser
                   85                  90                  95
   Pro Gln Asn Asn Pro Phe Thr Ala Val Leu Ser Gln Met Tyr Ala Gly
               100                 105                 110
   Trp Ala Gly Gly Met Gln Phe Arg Phe Ile Val Ala Gly Ser Gly Val
           115                 120                 125
   Phe Gly Gly Arg Leu Val Ala Ala Val Ile Pro Pro Gly Ile Glu Ile
       130                 135                 140
   Gly Pro Gly Leu Glu Val Arg Gln Phe Pro His Val Val Ile Asp Ala
   145                 150                 155                 160
   Arg Ser Leu Glu Pro Val Thr Ile Thr Met Pro Asp Leu Arg Pro Asn
                   165                 170                 175
   Met Tyr His Pro Thr Gly Asn Pro Gly Leu Val Pro Thr Leu Val Leu
               180                 185                 190
   Ser Val Tyr Asn Asn Leu Ile Asn Pro Phe Gly Gly Ser Thr Ser Ala
           195                 200                 205
   Ile Gln Val Thr Val Glu Thr Arg Pro Ser Glu Asp Phe Glu Phe Val
       210                 215                 220
   Met Ile Arg Ala Pro Ser Ser Lys Thr Val Asp Ser Ile Ser Pro Ala
   225                 230                 235                 240
   Asp Leu Leu Thr Thr Pro Val Leu Thr Gly Val Gly Thr Asp Asn Arg
                   245                 250                 255
   Trp Asn Gly Glu Ile Val Gly Leu Gln Pro Val Pro Gly Gly Phe Ser
               260                 265                 270
   Thr Cys Asn Arg His Trp Asn Leu Asn Gly Ser Thr Phe Gly Trp Ser
           275                 280                 285
   Ser Pro Arg Phe Ala Ala Ile Asp His Asp Arg Gly Asn Ala Ser Tyr
       290                 295                 300
   Pro Gly Ser Ser Ser Ser Asn Val Leu Glu Leu Trp Tyr Ala Ser Ala
   305                 310                 315                 320
   Gly Ser Ala Ala Asp Asn Pro Ile Ser Gln Ile Ala Pro Asp Gly Phe
                   325                 330                 335
   Pro Asp Met Ser Phe Val Pro Phe Ser Gly Thr Thr Val Pro Thr Ala
               340                 345                 350
   Gly Trp Val Gly Phe Gly Gly Ile Trp Asn Ser Ser Asn Gly Ala Pro
           355                 360                 365
   Phe Val Thr Thr Val Gln Ala Tyr Glu Leu Gly Phe Ala Thr Gly Ala
       370                 375                 380
   Pro Ser Asn Pro Gln Pro Thr Thr Thr Thr Ser Gly Ala Gln Ile Val
   385                 390                 395                 400
   Ala Lys Ser Ile Tyr Gly Val Ala Thr Gly Ile Asn Gln Ala Thr Ala
                   405                 410                 415
   Gly Leu Phe Val Met Ala Ser Gly Val Ile Ser Thr Pro Asn Ser Ser
               420                 425                 430
   Ala Ile Thr Tyr Thr Pro Gln Pro Asn Arg Ile Val Asn Ala Pro Gly
           435                 440                 445
   Thr Pro Ala Ala Ala Pro Ile Gly Lys Asn Thr Pro Ile Met Phe Ala
       450                 455                 460
   Ser Val Val Arg Arg Thr Gly Asp Ile Asn Ala Glu Ala Gly Ser Thr
   465                 470                 475                 480
   Asn Gly Thr Gln Tyr Gly Ala Gly Ser Gln Pro Leu Pro Val Thr Val
                   485                 490                 495
   Gly Leu Ser Leu Asn Asn Tyr Ser Ser Ala Leu Met Pro Gly Gln Phe
               500                 505                 510
   Phe Val Trp Gln Leu Asn Phe Ala Ser Gly Phe Met Glu Leu Gly Leu
           515                 520                 525
   Ser Val Asp Gly Tyr Phe Tyr Ala Gly Thr Gly Ala Ser Ala Thr Leu
       530                 535                 540
   Ile Asp Leu Ser Glu Leu Val Asp Ile Arg Pro Val Gly Pro Arg Pro
   545                 550                 555                 560
   Ser Thr Ser Thr Leu Val Tyr Asn Leu Gly Gly Thr Thr Asn Gly Phe
                   565                 570                 575
   Ser Tyr Val
<210> 4
   <211> 579
   <212> PRT
   <213> Artificial Sequence
<220>
   <223> VP1 of RHDVa
<400> 4
   Met Glu Gly Lys Ala Arg Thr Ala Pro Gln Gly Glu Ala Ala Gly Thr
   1               5                   10                  15
   Ala Thr Thr Ala Ser Val Pro Gly Thr Thr Thr Asp Gly Met Asp Pro
               20                  25                  30
   Gly Val Val Ala Thr Thr Ser Val Val Thr Ala Glu Asn Ser Ser Ala
           35                  40                  45
   Ser Ile Ala Thr Ala Gly Ile Gly Gly Pro Pro Gln Gln Val Asp Gln
       50                  55                  60
   Gln Glu Thr Trp Arg Thr Asn Phe Tyr Tyr Asn Asp Val Phe Thr Trp
   65                  70                  75                  80
   Ser Val Ala Asp Ala Pro Gly Ser Ile Leu Tyr Thr Val Gln His Ser
                   85                  90                  95
   Pro Gln Asn Asn Pro Phe Thr Ala Val Leu Ser Gln Met Tyr Ala Gly
               100                 105                 110
   Trp Ala Gly Gly Met Gln Phe Arg Phe Ile Val Ala Gly Ser Gly Val
           115                 120                 125
   Phe Gly Gly Arg Leu Val Ala Ala Val Ile Pro Pro Gly Ile Glu Ile
       130                 135                 140
   Gly Pro Gly Leu Glu Val Arg Gln Phe Pro His Val Val Ile Asp Ala
   145                 150                 155                 160
   Arg Ser Leu Glu Pro Val Thr Ile Thr Met Pro Asp Leu Arg Pro Asn
                   165                 170                 175
   Met Tyr His Pro Thr Gly Asp Pro Gly Leu Val Pro Thr Leu Val Leu
               180                 185                 190
   Ser Val Tyr Asn Asn Leu Ile Asn Pro Phe Gly Gly Ser Thr Ser Ala
           195                 200                 205
   Ile Gln Val Thr Val Glu Thr Arg Pro Ser Glu Asp Phe Glu Phe Val
       210                 215                 220
   Met Ile Arg Ala Pro Ser Ser Lys Thr Val Asp Ser Ile Ser Pro Ala
   225                 230                 235                 240
   Gly Leu Leu Thr Thr Pro Val Leu Thr Gly Val Gly Asn Asp Asn Arg
                   245                 250                 255
   Trp Asn Gly Gln Ile Val Gly Leu Gln Pro Val Pro Gly Gly Phe Ser
               260                 265                 270
   Thr Cys Asn Arg His Trp Asn Leu Asn Gly Ser Thr Tyr Gly Trp Ser
           275                 280                 285
   Ser Pro Arg Phe Ala Asp Ile Asp His Arg Arg Gly Ser Ala Ser Tyr
       290                 295                 300
   Pro Gly Ser Asn Ala Thr Asn Val Leu Gln Phe Trp Tyr Ala Asn Ala
   305                 310                 315                 320
   Gly Ser Ala Ile Asp Asn Pro Ile Ser Gln Val Ala Pro Asp Gly Phe
                   325                 330                 335
   Pro Asp Met Ser Phe Val Pro Phe Asn Gly Pro Gly Ile Pro Ala Ala
               340                 345                 350
   Gly Trp Val Gly Phe Gly Ala Ile Trp Asn Ser Asn Ser Gly Ala Pro
           355                 360                 365
   Asn Val Thr Thr Val Gln Ala Tyr Glu Leu Gly Phe Ala Thr Gly Ala
       370                 375                 380
   Pro Gly Asn Leu Gln Pro Thr Thr Asn Thr Ser Gly Ser Gln Thr Val
   385                 390                 395                 400
   Ala Lys Ser Ile Tyr Ala Val Val Thr Gly Thr Ala Gln Asn Pro Ala
                   405                 410                 415
   Gly Leu Phe Val Met Ala Ser Gly Val Ile Ser Thr Pro Ser Ala Asn
               420                 425                 430
   Ala Ile Thr Tyr Thr Pro Gln Pro Asp Arg Ile Val Thr Thr Pro Gly
           435                 440                 445
   Thr Pro Ala Ala Ala Pro Val Gly Lys Asn Thr Pro Ile Met Phe Ala
       450                 455                 460
   Ser Val Val Arg Arg Thr Gly Asp Val Asn Ala Thr Ala Gly Ser Ala
   465                 470                 475                 480
   Asn Gly Thr Gln Tyr Gly Thr Gly Ser Gln Pro Leu Pro Val Thr Ile
                   485                 490                 495
   Gly Leu Ser Leu Asn Asn Tyr Ser Ser Ala Leu Met Pro Gly Gln Phe
               500                 505                 510
   Phe Val Trp Gln Leu Thr Phe Ala Ser Gly Phe Met Glu Ile Gly Leu
           515                 520                 525
   Ser Val Asp Gly Tyr Phe Tyr Ala Gly Thr Gly Ala Ser Thr Thr Leu
       530                 535                 540
   Ile Asp Leu Thr Glu Leu Ile Asp Val Arg Pro Val Gly Pro Arg Pro
   545                 550                 555                 560
   Ser Lys Ser Thr Leu Val Phe Asn Leu Gly Gly Thr Ala Asn Gly Phe
                   565                 570                 575
   Ser Tyr Val
<210> 5
   <211> 579
   <212> PRT
   <213> Artificial Sequence
<220>
   <223> VP1 of RHDVb
<400> 5
   Met Glu Gly Lys Ala Arg Thr Ala Ser Gln Gly Glu Thr Ala Gly Thr
   1               5                   10                  15
   Ala Thr Thr Ala Ser Val Pro Gly Thr Thr Thr Asp Gly Met Asp Pro
               20                  25                  30
   Gly Val Val Ala Thr Thr Ser Val Val Thr Thr Glu Asn Ala Ser Thr
           35                  40                  45
   Ser Ile Ala Thr Ala Gly Ile Gly Gly Pro Pro Gln Gln Val Asp Gln
       50                  55                  60
   Gln Glu Thr Trp Arg Thr Asn Phe Tyr Tyr Asn Asp Val Phe Thr Trp
   65                  70                  75                  80
   Ser Val Ala Asp Ala Pro Gly Asn Ile Leu Tyr Thr Val Gln His Ser
                   85                  90                  95
   Pro Gln Asn Asn Pro Phe Thr Ala Val Leu Ser Gln Met Tyr Ala Gly
               100                 105                 110
   Trp Ala Gly Gly Met Gln Phe Arg Phe Ile Val Ala Gly Ser Gly Val
           115                 120                 125
   Phe Gly Gly Arg Leu Val Ala Ala Val Ile Pro Pro Gly Ile Glu Ile
       130                 135                 140
   Gly Pro Gly Leu Glu Val Arg Gln Phe Pro His Val Val Ile Asp Ala
   145                 150                 155                 160
   Arg Ser Leu Glu Pro Val Thr Ile Thr Met Pro Asp Leu Arg Pro Asn
                   165                 170                 175
   Met Tyr His Pro Thr Gly Asn Pro Gly Leu Val Pro Thr Leu Val Leu
               180                 185                 190
   Ser Val Tyr Asn Asn Leu Ile Asn Pro Phe Gly Gly Ser Thr Ser Ala
           195                 200                 205
   Ile Gln Val Thr Val Glu Thr Arg Pro Ser Glu Asp Phe Glu Phe Val
       210                 215                 220
   Met Ile Arg Ala Pro Ser Ser Lys Thr Val Asp Ser Ile Ser Pro Ala
   225                 230                 235                 240
   Asp Leu Leu Thr Thr Pro Val Leu Thr Gly Val Gly Thr Asp Asn Arg
                   245                 250                 255
   Trp Asn Gly Glu Ile Val Gly Leu Gln Pro Val Pro Gly Gly Phe Ser
               260                 265                 270
   Thr Cys Asn Arg His Trp Asn Leu Asn Gly Ser Thr Phe Gly Trp Ser
           275                 280                 285
   Ser Pro Arg Phe Ala Ala Ile Asp His Asp Arg Gly Asn Ala Ser Tyr
       290                 295                 300
   Pro Gly Ser Ser Ser Ser Asn Val Leu Glu Leu Trp Tyr Ala Ser Ala
   305                 310                 315                 320
   Gly Ser Ala Ala Asp Asn Pro Ile Ser Gln Ile Ala Pro Asp Gly Phe
                   325                 330                 335
   Pro Asp Met Ser Phe Val Pro Phe Ser Gly Thr Thr Val Pro Thr Ala
               340                 345                 350
   Gly Trp Val Gly Phe Gly Gly Ile Trp Asn Ser Ser Asn Gly Ala Pro
           355                 360                 365
   Phe Val Thr Thr Val Gln Ala Tyr Glu Leu Gly Phe Ala Thr Gly Ala
       370                 375                 380
   Pro Ser Asn Pro Gln Pro Thr Thr Thr Thr Ser Gly Ala Gln Ile Val
   385                 390                 395                 400
   Ala Lys Ser Ile Tyr Gly Val Ala Thr Gly Ile Asn Gln Ala Thr Ala
                   405                 410                 415
   Gly Leu Phe Val Met Ala Ser Gly Val Ile Ser Thr Pro Asn Ser Ser
               420                 425                 430
   Ala Ile Thr Tyr Thr Pro Gln Pro Asn Arg Ile Val Asn Ala Pro Gly
           435                 440                 445
   Thr Pro Ala Ala Ala Pro Ile Gly Lys Asn Thr Pro Ile Met Phe Ala
       450                 455                 460
   Ser Val Val Arg Arg Thr Gly Asp Ile Asn Ala Glu Ala Gly Ser Thr
   465                 470                 475                 480
   Asn Gly Thr Gln Tyr Gly Ala Gly Ser Gln Pro Leu Pro Val Thr Val
                   485                 490                 495
   Gly Leu Ser Leu Asn Asn Tyr Ser Ser Ala Leu Met Pro Gly Gln Phe
               500                 505                 510
   Phe Val Trp Gln Leu Asn Phe Ala Ser Gly Phe Met Glu Leu Gly Leu
           515                 520                 525
   Ser Val Asp Gly Tyr Phe Tyr Ala Gly Thr Gly Ala Ser Ala Thr Leu
       530                 535                 540
   Ile Asp Leu Ser Glu Leu Val Asp Ile Arg Pro Val Gly Pro Arg Pro
   545                 550                 555                 560
   Ser Thr Ser Thr Leu Val Tyr Asn Leu Gly Gly Thr Thr Asn Gly Phe
                   565                 570                 575
   Ser Tyr Val
<210> 6
   <211> 1743
   <212> DNA
   <213> Artificial Sequence
<220>
   <223> Sequence encoding RHDVab chimera VP1 protein
<400> 6
   atggagggca aagcccgcac agcgccgcaa ggcgaagcag caggcactgc caccacagca      60
   tcagtccctg gaaccacaac cgatggcatg gaccccggcg ttgtggccac taccagcgtg     120
   gtcactgcag agaattcatc cgcatcgatt gccactgcag gaattggtgg ccctccccag     180
   caagtggatc agcaagaaac atggaggacg aacttctact acaacgacgt tttcacctgg     240
   agcgtggcgg atgctcctgg taacatcctg tacaccgtgc agcactcgcc tcaaaacaac     300
   cccttcactg ctgtcctctc ccagatgtac gccggctggg caggtggtat gcaattcagg     360
   ttcatcgtcg ccggttcagg cgttttcggc ggaagactcg tggctgccgt cattccaccg     420
   ggcatcgaga ttggaccggg tttggaagtg aggcagttcc cacacgtcgt tatcgacgcc     480
   agatccctgg agcctgtcac aattacgatg ccagatttgc gcccgaacat gtaccatcct     540
   accggcaacc ccggattggt cccaactttg gttctgtcag tgtacaacaa cctgatcaac     600
   cccttcggtg gcagtacctc ggctattcag gttaccgtgg agactcgccc tagtgaggac     660
   ttcgaattcg tcatgatccg tgccccctcc agcaagactg ttgactccat tagcccggca     720
   gatctgctca ccactcctgt gttgacaggc gtcggaacgg ataaccgctg gaacggagaa     780
   atcgtgggtc tgcagcctgt ccccggaggt ttcagcacat gcaaccgtca ctggaacctc     840
   aacggctcta ccttcggatg gtcttcacca cgcttcgcag cgatcgacca tgatcgtggt     900
   aacgctagct accctggcag ttcgtccagc aacgtgctgg agttgtggta cgcctctgca     960
   ggttcagctg ccgacaaccc catctctcag attgctccgg acggtttccc tgatatgtcc    1020
   ttcgtcccct tcagcggcac aacggtgcca acagctggtt gggtcggctt cggcggaatc    1080
   tggaactctt caaacggagc gcccttcgtc accactgttc aagcttacga actgggtttc    1140
   gcgactggcg ctccatcaaa cccacagccg acaacgacca ctagtggtgc ccaaatcgtt    1200
   gcaaagtcga tttacggagt ggctacaggt atcaaccagg cgacggctgg actgttcgtt    1260
   atggcgagtg gtgtgatctc gacccctaac agttcggcta ttacctacac tcctcaaccc    1320
   aacaggatcg tgaacgcacc aggaacacct gcagctgctc ccatcggaaa aaacacgcca    1380
   attatgttcg cttccgtggt ccgccgtact ggcgacatca acgccgaagc aggctctaca    1440
   aacggaacgc agtacggtgc tggctcacaa ccactgccgg tcacagttgg actgagtctc    1500
   aacaactact ccagcgctct gatgccagga cagttcttcg tgtggcaact caacttcgct    1560
   tccggtttca tggagttggg cctgagcgtc gacggatact tctacgccgg aaccggtgcg    1620
   tctgctactc tcatcgactt gtcagaactg gtcgatatta ggcctgttgg tcctagaccc    1680
   agcacgagca ctctggtcta caacttgggc ggcactacta acggattctc atacgtctaa    1740
   taa                                                                  1743
<210> 7
   <211> 29
   <212> **DNA**
   <213> Artificial Sequence
<220>
   <223> Primer
<400> 7
   actgctcgag ataaatatgg agggcaaag 29
<210> 8
   <211> 43
   <212> DNA
   <213> Artificial Sequence
<220>
   <223> Primer
<400> 8
   actgtctaga atagcttact ttaaactata aacccaatta aac 43

## Description

### Technical field

The present invention relates to new compositions and/or vaccines to be used in the field of veterinary science. In particular, the present invention provides means for preventing rabbit haemorrhagic disease.

### Background art

The rabbit haemorrhagic disease virus (RHDV) is the causative agent of a highly infectious and fatal disease affecting domestic and wild rabbits (Cooke, 2002. Rev Sci Tech. 21(2):347-58; Moss et al., 2002. J Gen Virol. 83(Pt 10):2461-7; Ohlinger et al., 1990. J Virol. 64(7):3331-6). It is considered to be the single most economically important disease in rabbits worldwide with serious economic effects on cuniculture (Cheng et al., 2013. Antiviral Res. 97(3):227-31). The disease was first reported in China in 1984 and subsequently spread to most European countries, Mexico and other areas (Nowotny et al., 1997. Arch Virol. 142(4):657-73). The etiological agent is the rabbit haemorrhagic disease virus (RHDV), a non-enveloped, icosahedral RNA virus, belonging to the Genus Lagovirus within the Caliciviridae (Parra & Prieto, 1990. J Virol. 64(8):4013-5). The positive-sense single-stranded RNA genome of RHDV is 7.5kb in length and encodes a polyprotein precursor from the open reading frame ORF1, which is finally cleaved into the 60kDa major capsid protein (VP1, also known as VP60) and several non-structural proteins (Meyers et al., 1991. Virology. 184(2):664-76; Meyers et al., 2000. Virology. 276(2):349-63). Additionally, VP1 may also be expressed from a subgenomic RNA of approximately 2.4kb (Boga et al., 1992. Virus Res. 26(1):33-40; Parra et al., 1993. Virus Res. 27(3):219-28). The virus capsid (approx. 40nm in diameter) comprises 180 copies (90 dimers) of VP1, arranged with T=3 symmetry to form 12 pentamers and 20 hexamers (Prasad et al., 1994. JMol Bio. 240(3):256-64).

To date, vaccination remains the most effective method of preventing RHD. Commercially available vaccines mostly consist of inactivated forms of the original pathogens (Arguello Villares, 1991. Rev Sci Tech. 10(2):459-80) obtained from the livers collected from artificially infected rabbits. Although highly effective for controlling RHD, these types of vaccines often raise safety concerns because of the potential spread of the virus during the process of viral inactivation (Perez-Filgueira et al., 2007. Virology. 364(2):422-30). Virus-like particles (VLPs) are supramolecular assemblages with welldefined geometry that mimic the overall structure of native virions, while lacking any viral genome. These multimeric protein cages are based on the natural intrinsic ability of structural viral subunits to spontaneously self-assemble into nanoparticles (in the range of 25-100 nm), when produced using recombinant expression systems. They are composed of multiple copies of one or more viral proteins and are usually antigenically indistinguishable from infectious viruses or subviral particles, having been recognized as safe and effective vaccine candidates that simultaneously lead to strong humoral and cellular immune responses (Chackerian, 2007. Expert Rev Vaccines. 6(3):381-90; Demi et al., 2005. Mol Immunol. 42(2):259-77; Grgacic & Anderson, 2006. Methods. 40(1):60-5; Kushnir et al., 2012. Vaccine. 31(1):58-83). Immunization with the VP1 capsid protein obtained from Escherichia coli (Boga et al., 1994. J Gen Virol. 75(Pt 9):2409-13), Saccharomyces cerevisiae (Boga et al., 1997. J Gen Virol. 78(Pt 9):2315-8), plants (Castanon et al., 1999. J Virol. 73(5):4452-5; Fernandez-Fernandez et al., 2001. Virology. 280(2):283-91), mammalian and insect cells using recombinant poxvirus-based (Bertagnoli et al., 1996. J Virol. 70(8):5061-6) and baculovirus-based systems (Laurent et al., 1994. J Virol. 68(10):6794-8; Marin et al., 1995. Virus Res. 39(2-3): 119-28; Nagesha et al., 1995. Arch Virol. 140(6):1095-108; Sibilia et al., 1995. J Virol. 69(9):5812-5) protects rabbits against a lethal RHDV challenge (Gao et al., 2013. J Vet Sci. 14(4):441-7; Yuan et al., 2013. Vet Microbiol. 164(1-2): 1-8). However, none of the vaccines targeting RHDV have been successfully commercialized due to low expression levels, high costs, and complex purification procedures.

In the course of its evolution, RHDV split into six genotypes (Kerr et al., 2009. J Virol. 83(23):12129-38), all highly pathogenic and virulent. Genotype 6 is the antigenic subtype (RHDVa) that became prevalent in certain countries, including the USA (McIntosh et al., 2007. Virol J. 4:96). In 2011 outbreaks of a new variant RHDVb causing mortalities in kits (baby rabbits) were reported on Spanish rabbit farms (Dalton et al., 2014. Vet Microbiol. 169(1-2):67-73). This new variant appears to be closely related to a French isolate described in 2010 (Le Gall-Recule et al., 2011. Virology. 410(2):395-402). The new variant RHDVb (Dalton et al., 2012. Emerg Infect Dis. 18(12):2009-12), was purified from liver samples taken from previously vaccinated kits. This raises concerns as to whether the currently available vaccines will efficiently protect against variant RHDVb. In light of this and the immunogenic differences at the serotype level, there is an urgent need for the production of specific vaccines against the new strain. What is more, although the new variant has been the dominant virus causing RHD in most European countries in recent years, the fact that RHDVa still coexists in some areas makes it advisable to vaccinate animals against RHDVa and the new variant in dual formulations (Capucci et al., 2017. Vet Rec. 180(17):426).

WO 2017/046415 A2 discloses means and methods to increase the efficiency of recombinant protein expression. Several different sequences of the VP60 protein from different RHDVs are disclosed therein.

Pacho et al., 2016. Viral Immunol. 29(10):583-585 discloses the evaluation of a vaccine against RHDVb in rabbits.

The present invention aims to produce a stable vaccine for the prevention of RHDVb-associated and other possible future subtypes of RHDV-associated rabbit haemorrhagic disease which does not carry the risks associated with using inactivated virulent vaccines.

### Figures

**Figure 1****:** Diagram of an embodiment of the fusion protein of the present invention. VP1 chimeric sequence containing the 50 N-terminal amino acids of RHDV strain A (AST89) and the other 529 amino acids from RHDV strain B (N11).

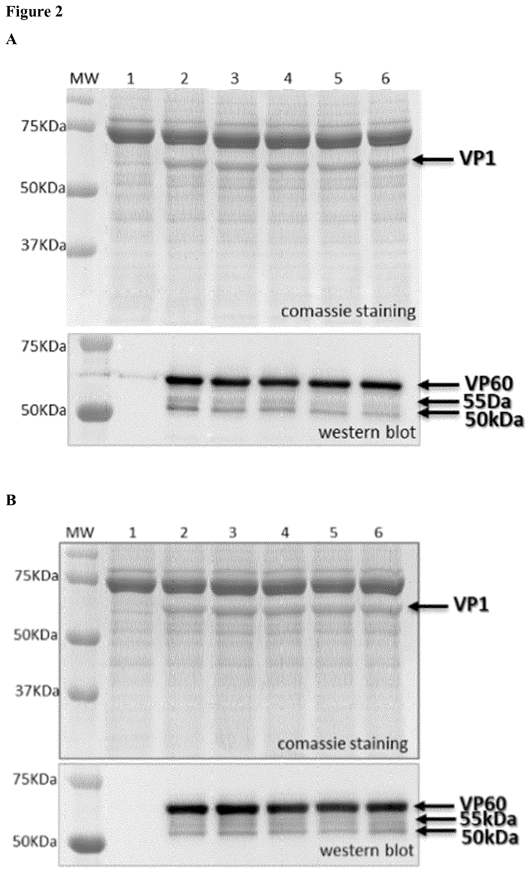

**Figure 2****:** SDS-PAGE and Western Blot analysis of (A) RHDVa VP1 and (B) RHDVb VP1 production in insects under different conditions. Different doses of virus and times of incubation before harvest were tested (lanes 2 to 6). A negative control consisting in an extract of pupae infected with non-recombinant baculovirus (BV) was also analysed (lane 1). Western Blot was performed using an anti-VP1 monoclonal antibody.
**Figure 3****:** Stability of RHDVa VP1 and RHDVb VP1. (A) SDS-PAGE of samples stored at 4 °C for 0, 1, 2 or 4 months. (B) Electron microscopy analysis by negative staining of the VLPs obtained from RHDVa VP1 and RHDVb VP1. (C) Three-dimensional maps of VP1 rRHDV T=3 and T=1 VLPs (obtained from Barcena et al., 2004. Virology. 322(1):118-34).
**Figure 4****:** Stability of the fusion protein RHDVab VP1. (A) SDS-PAGE of samples stored at 4 °C for 0, 1, 2 or 4 months. (B) Electron microscopy analysis by negative staining of the VLPs obtained from RHDVab VP1, RHDVa VP1 and RHDVb VP1.

### Summary of the invention

It has been described that for RHDV VLPs the charge configuration, which is modulated by pH among other factors, further regulates subunit interactions and hence T=3 versus T=1 assembly (T=1 assembly is less stable) (Barcena et al., 2004. Virology. 322(1):118-34). While developing the vaccine against RHDVb we found that these VLPs were less stable than those of RHDVa even if the same salt concentrations and pH levels were maintained. We hypothesized that the N-terminal sequence from RHDVb preferentially assembles into unstable T=1 than stable T=3 VLPs.

To test this hypothesis, we developed a chimeric VP1 protein with the C-terminal sequence from RHDVb, which comprises the majority of the antigenic sequences, and the N-terminal sequence from RHDVa. The resultant fusion protein was able to assemble into stable VLPs in the T=3 conformation. Moreover, the VLPs derived from this fusion protein were able to protect rabbits from a RHDVb challenge.

Thus, in a first aspect, the present invention provides a fusion protein comprising (i) an N-terminal arm (NTA) domain comprising SEQ ID NO: 1 or a sequence which has at least 98 % sequence identity with SEQ ID NO: 1, and (ii) a shell (S) domain and protrusion (P) domain comprising SEQ ID NO: 2 or a sequence which has at least 95 % sequence identity with SEQ ID NO: 2.

In a second aspect, the present invention provides a VLP comprising the fusion protein of the present invention.

In a third aspect, the present invention provides a composition comprising the VLP of the present invention.

In a fourth aspect, the present invention provides a pharmaceutical composition comprising the composition of the present invention and a pharmaceutically acceptable carrier and/or diluent.

In a fifth aspect, the present invention provides the fusion protein, VLP, composition or pharmaceutical composition of the present invention for use as a medicament.

In a sixth aspect, the present invention provides the fusion protein, VLP, composition or pharmaceutical composition of the present invention for use in the treatment and/or prevention of rabbit haemorrhagic disease.

In a seventh aspect, the present invention provides a nucleic acid encoding the fusion protein of the present invention.

In an eighth aspect, the present invention provides a vector comprising the nucleic acid of the present invention.

In a ninth aspect, the present invention provides a bacterial cell, yeast cell, mammalian cell, insect cell or insect comprising the nucleic acid of the present invention and/or the vector of the present invention.

Further aspects of the invention are provided in the claims

### Detailed description of the invention

### Definitions

As used herein, *"pharmaceutically acceptable carrier"* or *"pharmaceutically acceptable diluent"* means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and, without limiting the scope of the present invention, include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone. Other pharmaceutically acceptable carriers, excipients, or stabilizers, such as those described in Remington: The Science and Practice of Pharmacy 22nd edition, Pharmaceutical press (2012), ISBN-13: 9780857110626 may also be included.

The terms *"treatment"* and *"therapy",* as used in the present application, refer to a set of hygienic, pharmacological, surgical and/or physical means used with the intent to cure and/or alleviate a disease and/or symptoms with the goal of remediating the health problem. The terms *"treatment"* and *"therapy"* include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

The term *"prevention",* as used in the present application, refers to a set of hygienic, pharmacological, surgical and/or physical means used to prevent the onset and/or development of a disease and/or symptoms. The term *"prevention"* encompasses prophylactic methods, since these are used to maintain the health of an animal or individual.

The term *"therapeutically effective amount"* refers to an amount of fusion protein or VLPs which has a therapeutic effect and which is able to treat and/or prevent rabbit haemorrhagic disease.

The terms *"VP1"* and *"VP60"* refer to the major capsid protein of RHDV. The protein is subdivided into three domains: the N-terminal arm (NTA) domain, the shell (S) domain and the protrusion (P) domain. Examples of VP1 are the VP1 of RHDVa (SEQ ID NO: 4): and the VP1 of RHDVb (SEQ ID NO: 5):

In the present disclosure, the NTA domain of a VP1 protein starts at residue 1 and may end at any residue from 40-75 of the sequence. The S domain may then start at any residue from residue 40-75 and spans up to about residue 229 (Wang *et al.,* 2013. *PLos Pathog.* 9(1):e1003132). The P domain usually spans residues 238 to the C-terminal residue which is usually residue 579. In a preferred embodiment, the NTA domain of VP1 spans residues 1 to 50, and the S and P domain span residues 51-579.

The phrase *"VP1 protein derived from RHDVa"* refers to a protein which comprises the amino acid sequence of a VP1 protein found in RHDVa. The protein may further comprise modifications to simplify the downstream purification procedure such as affinity tags. The protein may also comprise other modifications such as post-translational modifications which may vary depending on the host that is used to express the amino acid sequence. These post-translational modifications may include glycosylation and proteolytic cleavage. The protein may also comprise modifications to increase the versatility of the system such as the inclusion of a portion of an isopeptide-forming protein (Brune et al., 2016. Sci Rep. 6:19234; Buldun et al., 2018. JAm Chem Soc. 140(8):3008-3018). The protein may also be chemically or enzymatically modified. For example, the protein may be PEGylated, fluorescently labelled or biotinylated using any means known in the art.

The phrase *"VP1 protein derived from RHDVb"* refers to a protein which comprises the amino acid sequence of a VP1 protein found in RHDVb. The protein may further comprise modifications to simplify the downstream purification procedure such as affinity tags. The protein may also comprise other modifications such as post-translational modifications which may vary depending on the host that is used to express the amino acid sequence. These post-translational modifications may include glycosylation and proteolytic cleavage. The protein may also comprise modifications to increase the versatility of the system such as the inclusion of a portion of an isopeptide-forming protein (Brune et al., 2016. Sci Rep. 6:19234; Buldun et al., 2018. JAm Chem Soc. 140(8):3008-3018). The protein may also be chemically or enzymatically modified. For example, the protein may be PEGylated, fluorescently labelled or biotinylated using any means known in the art.

The term *"vector"* refers to a vehicle or carrier which can carry foreign genetic material into a cell where it can be replicated and/or expressed. The term may include cloning vectors, expression vectors, transfer vectors and recombinant viruses.

The term *"sequence identity"* refers to a percentage value obtained when two sequences are compared using a pairwise sequence alignment tool. In the present case, the sequence identity is obtained using the global alignment tool *"EMBOSS Needle"* using the default settings (Rice et al., 2000. Trends Genet. 16(6):276-7; Li et al., 2015. Nucleic Acids Res. 43(W1):W580-4). The global alignment tool is available at: https://www.ebi.ac.uk/Tools/psa/.

The terms *"fusion protein"* and *"chimeric protein"* refer to a recombinant protein created by combining amino acid sequences of two or more proteins. The sequences may be fused together through a peptide bond (e.g. by creating a genetic construct wherein the two sequences are in the same open reading frame or by using split inteins), disulphide bond, thioester bond, chemical linker such as sulfosuccinimidyl 6-(3'-(2-pyridyldithio)propionamido)hexanoate, isopeptide bond or a carbodiimide. In a preferred embodiment, the sequences are fused together through a peptide bond, preferably by incorporating the two sequences in tandem in the same open reading frame.

The phrase *"rabbit haemorrhagic disease virus of a different subtype"* refers to any RHDV which is not RHDVa. In a preferred embodiment, the RHDV of a different subtype is RHDVb.

The term *"pharmaceutically acceptable adjuvant"* refers to any and all substances which enhance the body's immune response to an antigen. Non-limiting examples of pharmaceutically acceptable adjuvants are: Alum, Freund's Incomplete Adjuvant, MF59, carbomers, saponins, synthetic analogs of dsRNA such as poly(I:C), bacterial LPS, bacterial flagellin, imidazolquinolines, oligodeoxynucleotides containing specific CpG motifs, fragments of bacterial cell walls such as muramyl dipeptide and Quil-A.

### Fusion protein

In a first aspect, the present invention provides a fusion protein comprising: (i) an N-terminal arm (NTA) domain comprising SEQ ID NO: 1 or a sequence which has at least 98 % sequence identity with SEQ ID NO: 1; and (ii) a shell (S) domain and protrusion (P) domain comprising SEQ ID NO: 2 or a sequence which has at least 95 % sequence identity with SEQ ID NO: 2.

In a preferred embodiment, the NTA domain comprises SEQ ID NO: 1 or a sequence which has at least 99 % sequence identity with SEQ ID NO: 1.

SEQ ID NO: 1 refers to the following sequence derived from the VP1 of RHDVa:
MEGKARTAPQGEAAGTATTASVPGTTTDGMDPGVVATTSVVTAENSSASI

In a preferred embodiment, the S domain and P domain comprises SEQ ID NO: 2 or a sequence which has at least 97 % sequence identity with SEQ ID NO: 2. Preferably, the S domain and P domain comprises SEQ ID NO: 2 or a sequence which has at least 98 or 99 % sequence identity with SEQ ID NO: 2. More preferably, the S domain and P domain comprises SEQ ID NO: 2 or a sequence which has at least 99 % sequence identity with SEQ ID NO: 2.

SEQ ID NO: 2 refers to the following sequence derived from the VP1 of RHDVb:

In a preferred embodiment, the fusion protein comprises: (i) SEQ ID NO: 1 or a sequence which has at least 98 % sequence identity with SEQ ID NO: 1; and (ii) SEQ ID NO: 2 or a sequence which has at least 99 % sequence identity with SEQ ID NO: 2.

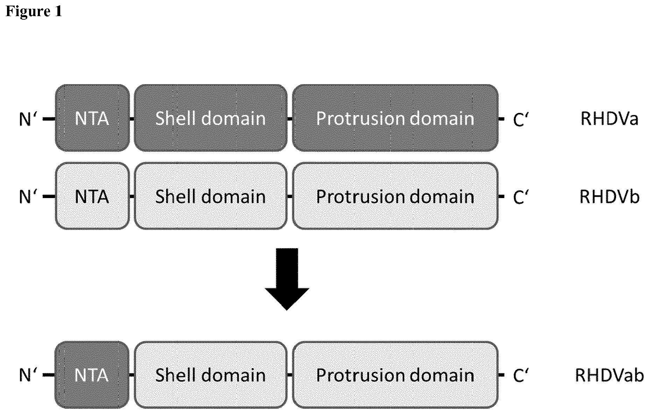

In a preferred embodiment, sequence (i) of the fusion protein is present in the N-terminal region and sequence (ii) is present in the C-terminal region. In other words, the two components are arranged so that the NTA is at the N-terminus and the S and P domain are at the C-terminus such as in the case of the RHDVab chimeric construct used in the Examples. This is further illustrated and exemplified in Figure 1.

In a preferred embodiment, the fusion protein comprises SEQ ID NO: 3 or a sequence which has at least 97 % sequence identity with SEQ ID NO: 3. Preferably, the fusion protein comprises SEQ ID NO: 3 or a sequence which has at least 98 or 99 % sequence identity with SEQ ID NO: 3. More preferably, the fusion protein comprises SEQ ID NO: 3 or a sequence which has at least 99 % sequence identity with SEQ ID NO: 3.

SEQ ID NO: 3 is the sequence of a chimeric protein which has the NTA domain of RHDVa and the S and P domains of RHDVb:

### VLP

The fusion protein of the present invention self-assembles into VLPs. Thus, in a second aspect, the present invention provides a VLP comprising the fusion protein of the present invention.

In a preferred embodiment, the VLP is composed entirely of the fusion protein of the present invention. In an alternative embodiment, the VLP further comprises a VP1 protein derived from a RHDV, preferably RHDVa. In other words, the VLP is composed of the fusion protein of the present invention as well as a VP1 protein derived from an RHDV, preferably RHDVa. Such a VLP could be obtained by expressing the fusion protein of the present invention and the VP1 of RHDVa in tandem in the same host.

In a preferred embodiment, in more than 50 % of the cases the VLP is assembled in a structure with T=3 symmetry. Preferably, in more than 55, 60, 65, 70, 75, 80, 85, 90 or 95 % of the cases the VLP is assembled in a structure with T=3 symmetry. More preferably, in more than 80 % of the cases the VLP is assembled in a structure with T=3 symmetry.

In a preferred embodiment, the VLP is modified to carry a payload. The payload may be a therapeutic molecule such as a small drug molecule or siRNA, or the payload may act as an adjuvant to increase the immune response towards the VLP. In a preferred embodiment, the VLP encapsulates a payload. In a preferred embodiment, the VLP forms the basis of a multipurpose vaccine (see, for example, Fernandez et al., 2013. PLoS One. 8(2):e56417).

In a preferred embodiment, the diameter of the VLP is 26 to 44 nm. Preferably, the diameter of the VLP is 35 to 44 nm.

The VLPs of the present invention may be expressed and purified using any of the methods known in the prior art (Boga et al., 1994. J Gen Virol. 75(Pt 9):2409-13; Boga et al., 1997. J Gen Virol. 78(Pt 9):2315-8; Castanon et al., 1999. J Virol. 73(5):4452-5; Fernandez-Fernandez et al., 2001. Virology. 280(2):283-91; Bertagnoli et al., 1996. J Virol. 70(8):5061-6; Laurent et al., 1994. J Virol. 68(10):6794-8; Marin et al., 1995. Virus Res. 39(2-3): 119-28; Nagesha et al., 1995. Arch Virol. 140(6):1095-108; Sibilia et al., 1995. J Virol. 69(9):5812-5; Fernandez et al., 2013. PLoS One. 8(2):e56417). In a preferred embodiment, the VLPs are expressed in a TopBac^{®} system (Algenex) in pupae (see WO 2017/046415 A2 and WO 2012/168492 A2).

### Compositions and pharmaceutical compositions

In a third aspect, the present invention provides a composition comprising the VLP of the present invention.

In a preferred embodiment, the composition comprises a plurality of VLPs and more than 50 % of the VLPs are assembled in a structure with T=3 symmetry. Preferably, more than 55, 60, 65, 70, 75, 80, 85, 90 or 95 % of the VLPs are assembled in a structure with T=3 symmetry. More preferably, more than 80 % of the VLPs are assembled in a structure with T=3 symmetry.

In a preferred embodiment, the composition further comprises a second VLP comprising a VP1 protein derived from RHDVa. In other words, the composition may comprise a VLP composed of the fusion protein of the present invention and a VLP composed of VP1 from RHDVa. In the examples of the present invention it was found that when a vaccine comprises RHDVab (VLPs composed of a fusion protein) and RHDVa (VLPs composed of VP1 from RHDVa), resistance was conferred against both RHDVa and RHDVb (see Example 4).

In a preferred embodiment, the composition further comprises a VLP comprising SEQ ID NO: 4 or a protein with 85 % sequence identity with SEQ ID NO: 4. Preferably, the composition further comprises a VLP comprising SEQ ID NO: 4 or a protein with 90, 95 or 99 % sequence identity with SEQ ID NO: 4.

In a preferred embodiment, the composition further comprises a VLP composed of SEQ ID NO: 4 or a protein with 85 % sequence identity with SEQ ID NO: 4. Preferably, the composition further comprises a VLP composed of SEQ ID NO: 4 or a protein with 90, 95 or 99 % sequence identity with SEQ ID NO: 4.

In an alternative aspect, the present invention provides a composition comprising the fusion protein of the present invention. In a preferred embodiment, the composition may further comprise the VP1 protein of RHDVa.

In a fourth aspect, the present invention provides a pharmaceutical composition comprising the composition of the present invention and a pharmaceutically acceptable carrier and/or diluent.

A pharmaceutical composition as described herein may also contain other substances. These substances include, but are not limited to, cryoprotectants, lyoprotectants, surfactants, bulking agents, anti-oxidants, and stabilizing agents. In some embodiments, the pharmaceutical composition may be lyophilized.

The term *"cryoprotectant"* as used herein, includes agents which provide stability to the VLP against freezing-induced stresses, by being preferentially excluded from the VLP's surface. Cryoprotectants may also offer protection during primary and secondary drying and long-term product storage. Non-limiting examples of cryoprotectants include sugars, such as sucrose, glucose, trehalose, mannitol, mannose, and lactose; polymers, such as dextran, hydroxyethyl starch and polyethylene glycol; surfactants, such as polysorbates (e.g., PS-20 or PS-80); and amino acids, such as glycine, arginine, leucine, and serine. A cryoprotectant exhibiting low toxicity in biological systems is generally used.

In one embodiment, a lyoprotectant is added to a pharmaceutical composition described herein. The term *"lyoprotectant"* as used herein, includes agents that provide stability to the VLP during the freeze-drying or dehydration process (primary and secondary freeze-drying cycles), by providing an amorphous glassy matrix and by binding with the VLP's surface through hydrogen bonding, replacing the water molecules that are removed during the drying process. This helps to minimize product degradation during the lyophilization cycle, and improve the long-term product stability. Non-limiting examples of lyoprotectants include sugars, such as sucrose or trehalose; an amino acid, such as monosodium glutamate, non-crystalline glycine or histidine; a methylamine, such as betaine; a lyotropic salt, such as magnesium sulfate; a polyol, such as trihydric or higher sugar alcohols, e.g., glycerin, erythritol, glycerol, arabitol, xylitol, sorbitol, and mannitol; propylene glycol; polyethylene glycol; pluronics; and combinations thereof. The amount of lyoprotectant added to a pharmaceutical composition is generally an amount that does not lead to an unacceptable amount of degradation of the VLP when the pharmaceutical composition is lyophilized.

In some embodiments, a bulking agent is included in the pharmaceutical composition. The term "bulking agent" as used herein, includes agents that provide the structure of the freeze- dried product without interacting directly with the pharmaceutical product. In addition to providing a pharmaceutically elegant cake, bulking agents may also impart useful qualities in regard to modifying the collapse temperature, providing freeze-thaw protection, and enhancing the VLP stability over long-term storage. Non-limiting examples of bulking agents include mannitol, glycine, lactose, and sucrose. Bulking agents may be crystalline (such as glycine, mannitol, or sodium chloride) or amorphous (such as dextran, hydroxyethyl starch) and are generally used in formulations in an amount from 0.5% to 10%.

Other pharmaceutically acceptable carriers, excipients, or stabilizers, such as those described in Remington's Pharmaceutical Sciences 16th edition, Osol, A. Ed. (1980) may also be included in a pharmaceutical composition described herein, provided that they do not adversely affect the desired characteristics of the pharmaceutical composition. As used herein, "pharmaceutically acceptable carrier" means any and all solvents, dispersion media, coatings, antibacterial and antifungal agents, isotonic and absorption delaying agents, compatible with pharmaceutical administration. The use of such media and agents for pharmaceutically active substances is well known in the art. Acceptable carriers, excipients, or stabilizers are nontoxic to recipients at the dosages and concentrations employed and include: additional buffering agents; preservatives; co-solvents; antioxidants, including ascorbic acid and methionine; chelating agents such as EDTA; metal complexes (e.g., Zn-protein complexes); biodegradable polymers, such as polyesters; salt-forming counterions, such as sodium, polyhydric sugar alcohols; amino acids, such as alanine, glycine, glutamine, asparagine, histidine, arginine, lysine, ornithine, leucine, 2-phenylalanine, glutamic acid, and threonine; organic sugars or sugar alcohols, such as lactitol, stachyose, mannose, sorbose, xylose, ribose, ribitol, myoinisitose, myoinisitol, galactose, galactitol, glycerol, cyclitols (e.g., inositol), polyethylene glycol; sulfur containing reducing agents, such as urea, glutathione, thioctic acid, sodium thioglycolate, thioglycerol, [alpha]-monothioglycerol, and sodium thio sulfate; low molecular weight proteins, such as human serum albumin, bovine serum albumin, gelatin, or other immunoglobulins; and hydrophilic polymers, such as polyvinylpyrrolidone.

In a preferred embodiment, the pharmaceutical composition further comprises a pharmaceutically acceptable adjuvant. Preferably, the adjuvant is selected from the list consisting of Alum, Freund's Incomplete Adjuvant, MF59^{®}, carbomers, saponines, synthetic analogs of dsRNA such as poly(I:C), bacterial LPS, bacterial flagellin, imidazolquinolines, oligodeoxynucleotides containing specific CpG motifs, fragments of bacterial cell walls such as muramyl dipeptide and Quil-A^{®}.

The pharmaceutical composition may be prepared for oral, sublingual, buccal, intravenous, intramuscular, subcutaneous, intradermal, intraperitoneal, conjunctival, rectal, transdermal, topical and/or inhalation-mediated administration. In a preferred embodiment, the pharmaceutical composition may be a solution which is suitable for intravenous, intramuscular, intradermal, conjunctival, transdermal, intraperitoneal and/or subcutaneous administration. In another embodiment, the pharmaceutical composition may be a solution which is suitable for sublingual, buccal and/or inhalation-mediated administration routes. In an alternative embodiment, the pharmaceutical composition may be an aerosol which is suitable for inhalation-mediated administration. In a preferred embodiment, the pharmaceutical composition may be prepared for subcutaneous, intradermal and/or intraperitoneal administration.

The pharmaceutical composition may further comprise common excipients and carriers which are known in the state of the art. For solid pharmaceutical compositions, conventional nontoxic solid carriers may be used which include, for example, pharmaceutical grades of mannitol, lactose, starch, magnesium stearate, sodium saccharin, talcum, cellulose, glucose, sucrose, magnesium carbonate, and the like. For solution for injection, the pharmaceutical composition may further comprise cryoprotectants, lyoprotectants, surfactants, bulking agents, anti-oxidants, stabilizing agents and pharmaceutically acceptable carriers. For aerosol administration, the pharmaceutical compositions are generally supplied in finely divided form along with a surfactant and propellant. The surfactant must, of course, be nontoxic, and is generally soluble in the propellant. Representative of such agents are the esters or partial esters of fatty acids containing from 6 to 22 carbon atoms, such as caproic, octanoic, lauric, palmitic, stearic, linoleic, linolenic, olesteric and oleic acids with an aliphatic polyhydric alcohol or its cyclic anhydride. Mixed esters, such as mixed or natural glycerides may be employed. A carrier can also be included, as desired, as with, e.g., lecithin for intranasal delivery. For suppositories, traditional binders and carriers may include, for example, polyalkalene glycols or triglycerides.

In a preferred embodiment, the pharmaceutical composition is a vaccine capable of inducing an immune response. The design of pharmaceutical compositions for vaccines is well established, and is described, for example, in Remington's Pharmaceutical Sciences, latest edition, Mack Publishing Co., Easton, PA, and in Plotkin and Orenstein's book entitled Vaccines, 4th Ed., Saunders, Philadelphia, PA (2004).

In a preferred embodiment, the pharmaceutical composition is administered subcutaneously or intradermally, preferably subcutaneously.

### Medical uses

In a fifth aspect, the present invention provides the fusion protein, VLP, composition or pharmaceutical composition of the present invention for use as a medicament. In a sixth aspect, the present invention provides the fusion protein, VLP, composition or pharmaceutical composition of the present invention for use in the treatment and/or prevention of rabbit haemorrhagic disease.

In a preferred embodiment, the fusion protein, VLP, composition or pharmaceutical composition is administered to a rabbit and/or a therapeutically effective amount of the fusion protein, VLP, composition or pharmaceutical composition is administered.

In a preferred embodiment, the causative agent of the rabbit haemorrhagic disease is RHDVa and/or RHDVb.

In a preferred embodiment, the fusion protein, VLP, composition or pharmaceutical composition is administered prophylactically and/or the fusion protein, VLP, composition or pharmaceutical composition composition is administered to a rabbit of the species *Oryctolagus cuniculus.*

In a preferred embodiment, the rabbit is administered at least 0.5 µg of the VLPs of the present invention. Preferably, the rabbit is administered at least 1, 1.5, 2, 2.5, 3, 4, 5, 6, 7, 8, 9, 10, 11, 12, 13, 14, 15, 16, 17, 18, 19 or 20 µg of the VLPs of the present invention.

In a preferred embodiment, the fusion protein, VLP, composition or pharmaceutical composition is administered once. In a preferred embodiment, the fusion protein, VLP, composition or pharmaceutical composition is administered more than once. In a preferred embodiment, the fusion protein, VLP, composition or pharmaceutical composition is administered at least once every six months, once a year, once every two years, once every three years or once every four years. In a preferred embodiment, the fusion protein, VLP, composition or pharmaceutical composition is administered as a booster shot.

### Nucleic acids and vectors

In a seventh aspect, the present invention provides a nucleic acid comprising a sequence which encodes the fusion protein of the present invention. In a preferred embodiment, the sequence is codon optimized to ensure high expression of the fusion protein. Methods for optimizing the codons of a genetic construct are known in the art (Quax et al., 2015. Mol Cell. 59(2): 149-61).

In a preferred embodiment, the nucleic acid comprises SEQ ID NO: 6. SEQ ID NO: 6 is a nucleic acid sequence that encodes a chimeric protein which has the NTA domain of RHDVa and the S and P domains of RHDVb:

In an eighth aspect, the present invention provides a vector comprising the nucleic acid of the present invention. In a preferred embodiment, the vector is selected from a list consisting of a cloning vector, expression vector, transfer vector and recombinant virus.

The term *"cloning vector"* refers to a small piece of DNA, taken from a virus, a plasmid, or the cell of a higher organism, that can be stably maintained in an organism, and into which a foreign DNA fragment can be inserted for cloning purposes. A cloning vector generally involves the presence of restriction sites, an origin of replication for bacterial propagation and a selectable marker.

The term *"expression vector"* refers to a small piece of DNA, taken from a virus, a plasmid, or the cell of a higher organism, that can be stably maintained in an organism, and into which a foreign DNA fragment can be inserted for protein expression purposes. The expression vector can commandeer the host cell's mechanism for protein synthesis to produce the fusion protein encoded by the nucleic acid.

The term "transfer *vector"* refers to a small piece of DNA that allows for the insertion of a foreign DNA fragment into the genome of a recombinant virus.

The term *"recombinant virus"* refers to an infective agent that typically consists of a nucleic acid molecule in a protein coat, is too small to be seen by light microscopy, and is able to multiply only within the living cells of a host. The term also comprises recombinant bacteriophages and recombinant baculoviruses.

In a preferred embodiment, the vector is lyophilized or cryopreserved.

In a ninth aspect, the present invention provides a bacterial cell, yeast cell, mammalian cell, insect cell or insect comprising the nucleic acid of the present invention and/or the vector of the present invention. In a preferred embodiment, the bacterial cell, yeast cell, mammalian cell, insect cell or insect is cryopreserved.

### Examples

### Example 1: production of VLPs

In the case of RHDVa, the VP60 (also known as VP1) sequence was amplified from a donor plasmid *pVP60306GS* (López-Vidal et al., 2015. PLoS One. 10(10):e0140039) by PCR using the primers 5' ACTGCTCGAGATAAATATGGAGGGCAAAG (SEQ ID NO: 7) and 5' ACTGTCTAGAATAGCTTACTTTAAACTATAAACCCAATTAAAC (SEQ ID NO: 8). The amplified sequence was cloned into the intermediate plasmid TB3 (Gomez-Sebastian et al., 2014. PLoS One. 9(5):e96562) using the restriction sites XhoI and XbaI at 5'and 3'end respectively. Then the VP60 insert was digested using XhoI and PvuII and the insert was subcloned into the vector pFastBacTB3 (Gomez-Sebastian et al., 2014. PLoS One. 9(5):e96562) which had already been digested with XhoI and PmeI at the 5' and 3' end respectively. For RHDVb and RHDVab the sequences were codon optimized for expression in insect cells and were synthesized (SEQ ID NO: 6 was the sequence of the codon optimized fusion protein). The construct was designed to included restriction sites for NcoI and XhoI at 5'and 3'end respectively. The synthesized inserts were digested and cloned into a pFastBacTB3 plasmid digested with the same restriction enzymes.

The resulting plasmids were named pFBTB3-RHDVa, pFBTB3-RHDVb and pFBTB3-RHDVab, and were used to generate the recombinant baculoviruses using the Bac-to-Bac^{®} baculovirus expression system (Invitrogen, USA), following the manufacturer's instructions. The baculovirus vector BacNI (a baculovirus with no foreign gene) was used as a control. The baculovirus produced were subjected to two rounds of clonal selection by plaque formation in attached Sf9-RVN insect cells grown in semisolid SF900-III medium. Cloned recombinant baculoviruses were propagated and amplified in Sf9-RVN insect cells grown in suspension in ESF921 medium, in the absence of FBS, to reach infective titres of approximately 10⁸ pfu (plaque-forming units)/ml. Baculovirus stocks were stored at -80 °C in the presence of 5% glycerol.

*Trichoplusia ni* were reared following a previously described protocol (Barderas et al., 2000. J Virol Methods. 89(1-2): 129-36). Briefly, eggs were placed into larvae developmental cages, containing an artificial insect diet (Medin et al., 1995. Method Mol Biol. 39:265-75). The eggs hatched and larvae went through the 5 instar stages and then pupated. Silk is then removed from the pupae (see WO 2017/046415 A2) and each is injected with 5 µl of recombinant baculovirus diluted to reach the number of pfu per dose selected. To determine the optimal dose of recombinant baculovirus, groups of 50 pupae were infected with 5×10⁵, 5×10⁴ or 5×10³ pfu and were processed at 4 to 7 days postinfection (dpi) after incubation at temperatures of 26-28 °C. The collected pupae were weighed to obtain the total biomass and were frozen immediately and stored at -20 °C until they were processed for recombinant protein extraction. The total productivity yields for each condition was analysed and compared by SDS-PAGE and densitometry.

The crude protein extracts were obtained by homogenizing the frozen biomass in the adequate protein extraction buffer. The resulting suspension was subject to a conventional downstream process of centrifugation tangential flow filtration and chromatography to obtain the final product. An example of a conventional downstream process which could be used is disclosed in Fernandez et al., 2013. PLoS One. 8(2):e56417. The amount of recombinant protein was quantified by densitometry on SDS-PAGE using a BSA standard curve. The samples were stored at 4 °C before they were used to formulate the vaccines and immunize the animals.

### Example 2: production of RHDVa and RHDVb VLPs and stability

Experiments were carried out to establish optimal conditions for the maximum productivity of rVP60 in *T. ni* pupae. Pupae were inoculated with different doses of rRHDVa or rRHDVb virus and samples were processed at different times post-inoculation and analysed by SDS-PAGE and Western blot using an anti-VPl monoclonal antibody (INGENASA, Spain; Ref. ref.: M.17.RHD.I1A2). As previously described (Perez-Filgueira et al., 2007. Virology. 364(2):422-30), a pattern of three specific bands is shown when VP1 is expressed in insects; a major 60kDa band (VP60) and two minor bands of 55 and 50kDa approximately, probably as the result of partial proteolytic processing of the rVP60. The best conditions for maximum productivity were selected for each case.

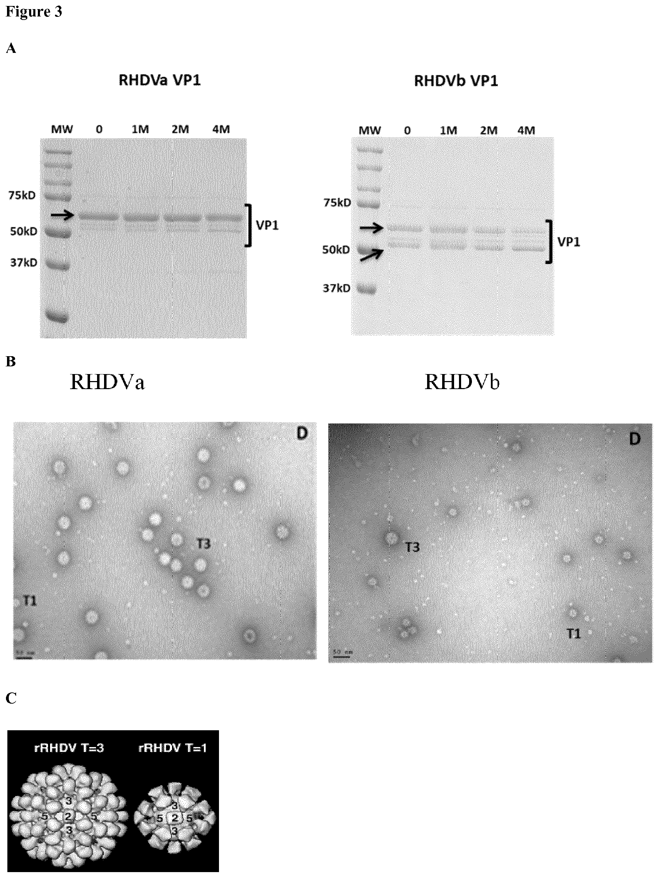

In order to analyse the stability of the VLPs produced in insects, the VLPs were analysed by SDS-PAGE after 1, 2 or 4 months of incubation at 4 °C. As shown in Figure 3, in the case of RHDVa the VP60 band was the most abundant specific band observed at time 0, and the described pattern of two minor specific lower bands (55 and 50kDa) was also observed. This pattern was shown to be maintained throughout the incubation, indicating that the protein is quite stable. In the case of RHDVb, after only 1 month of incubation at 4 °C there was a decrease in the intensity of the VP60 band along with an increase of the 50kDa band, indicating that the protein was being cleaved (Figure 3). This proteolysis gradually increased over time, indicating that the recombinant protein produced with RHDVb rBV (recombinant baculovirus) was not very stable.

We next analysed the two final products, RHDVa and RHDVb, by electron microscopy and negative staining. The images (Figure 3B) clearly show that T=3 structured VLPs had been assembled for the RHDVa samples, while in the case of RHDVb there was a predominance of T=1 VLPs. As it has been described before in the literature (Barcena et al., 2004. Virology. 322(1):118-34) the T=1 RHDV VLPs tend to be more unstable than T=3 VLPs, which correlates with the observed proteolysis in the case of strain/subtype B.

### Example 3: stability of the VLPs obtained using the fusion protein

The stability of the VLPs obtained using the chimeric VP1 protein was studied using the same methods described in Example 2.

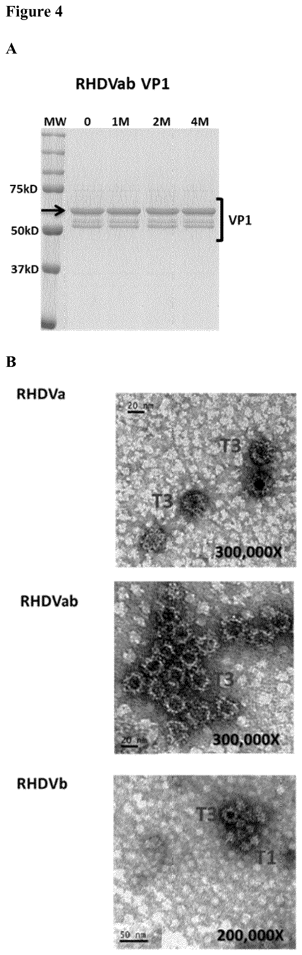

It was observed that the chimeric VP 1 protein, even after 4 months of incubation at 4 °C, maintained the proportions of the three bands in a similar manner as seen for RHDVa (Figures 3 and 4). This absence of proteolysis correlated with a predominance of T=3 structure VLPs observed by electron microscopy. These results indicate that the fusion protein does not undergo proteolysis during storage and the fusion protein assembles into stable VLPs.

Thus, we have developed a VLP-based vaccine against RHDVb which overcomes the instability problems intrinsic to the VLPs obtained using the VP1 protein of the wild-type RHDVb.

### Example 4: rabbit immunization using the VLPs

Once the problem of instability of the VLPs produced from the RHDVb strain was solved, we next performed protective efficacy tests in rabbits in order to determine if a dual vaccine formulated with both of the antigens produced, RHDVa and RHDVab, would protect from a challenge with either strain (RHDVa or virulent RHDVb). Different doses were tested in both cases and then the experiments were repeated with the selected dose.

### Test 1-RHDVb challenge

The protective efficacy of the dual vaccine containing the VLPs/VP1 of the invention was tested against virulent RHDVb. The vaccine formulation containing a dose of between 2 to 20 micrograms of each VLP (RHDVa and RHDVab) was tested against virulent RHDVb. A total of 24 New Zealand White rabbits of 30 days of age were distributed into 2 experimental groups comprising 12 subjects each. 1 group was vaccinated subcutaneously and the other group acted as an unvaccinated control.

All rabbits were challenged 7 days after vaccination with a virulent strain of RHDVb (Gal08/13) with a 100 LD₅₀ virus dose (see Duran Ferrer et al., 2015. Virologia: Publication Oficial de la Sociedad Española de Virologia. 18(1):78-79; ISSN: 2172-6523). Mortality and clinical signs were evaluated during the challenge. All of the vaccinated rabbits survived after the challenge, while all of the unvaccinated animals in the control group died. 11 died within 43 hours after the challenge and 1 died 9 days after the challenge.

qRT-PCR was then used to assess the presence of RHDVb in the liver. In all of the vaccinated rabbits no RHDVb RNA was found in the liver at day 15 after challenge. Conversely, all unvaccinated rabbits in the control group had high levels of RHDVb RNA in the liver.

### Test 2 - RHDVa challenge

The protective efficacy of the vaccine containing the VLPs/VP1 of the invention was tested against virulent RHDVa. A total of 34 New Zealand White rabbits were distributed into 3 experimental groups. Vaccinated animals received subcutaneously a dose of between 2 to 20 micrograms of each VLP (RHDVa and RHDVab).

1 group of 12 rabbits was vaccinated subcutaneously at 30 days of age (group 1), 1 group of 12 rabbits was vaccinated subcutaneously at 51 days of age (group 2) and the third group consisting of 10 unvaccinated rabbits acted as a control of challenge infection.

All rabbits were challenged at 58 days of age (4 and 1 weeks after vaccination of group 1 and group 2, respectively) with a 100 LD₅₀ dose of a virulent strain of RHDVa (Ast89) according to a consolidated challenge protocol (see Prieto et al., 2000. Res VetSci. 68(2): 181-7). Mortality and clinical signs were evaluated during the challenge.

All of the animals that were vaccinated at 30 days of age survived the challenge. 92 % of the animals that were vaccinated at 51 days of age survived the challenge. 90 % of the animals in the unvaccinated control group died within 48 hours after the challenge.

qRT-PCR was then used to assess the presence of RHDVa in the liver as it was done in the challenge experiment with RHDVb. In none of the rabbits vaccinated at 30 days of age RHDVa viral RNA was found in the liver at 15 days after challenge, while in more 75% of liver samples from rabbits vaccinated at 51 days RHDV RNA was found 15 days after challenge. Conversely, all unvaccinated rabbits in the control group had high levels of RHDVa RNA in the liver.

## Claims

1. A fusion protein comprising:
(i) an N-terminal arm (NTA) domain comprising SEQ ID NO: 1 or a sequence which has at least 98 % sequence identity with SEQ ID NO: 1; and
(ii) a shell (S) domain and protrusion (P) domain comprising SEQ ID NO: 2 or a sequence which has at least 95 % sequence identity with SEQ ID NO: 2.

2. The fusion protein according to claim 1, wherein the fusion protein comprises:
(i) SEQ ID NO: 1; and
(ii) SEQ ID NO: 2 or a sequence which has at least 99 % sequence identity with SEQ ID NO: 2.

3. The fusion protein according to any one of claims 1 to 2, wherein the fusion protein comprises SEQ ID NO: 3.

4. A virus-like particle (VLP) comprising the fusion protein according to any one of claims 1 to 3.

5. A composition comprising the VLP according to claim 4.

6. The composition according to claim 5, wherein the composition comprises a plurality of VLPs and more than 50% of the VLPs are assembled in a structure with T=3 symmetry.

7. The composition according to claim 5, wherein the composition further comprises a second VLP comprising a VP1 protein derived from RHDVa.

8. A pharmaceutical composition comprising the composition according to any one of claims 5 to 7 and a pharmaceutically acceptable carrier and/or diluent.

9. A nucleic acid comprising a sequence which encodes the fusion protein according to any one of claims 1 to 3.

10. A vector comprising the nucleic acid according to claim 9, preferably the vector is selected from a list consisting of a cloning vector, expression vector, transfer vector and recombinant virus.

11. A bacterial cell, yeast cell, mammalian cell, insect cell or insect comprising the nucleic acid according to claim 9 and/or the vector according to claim 10.

12. The fusion protein according to any one of claims 1 to 3, the VLP according to claim 4, the composition according to any one of claims 5 to 7 or the pharmaceutical composition according to claim 8 for use as a medicament.

13. The fusion protein according to any one of claims 1 to 3, the VLP according to claim 4, the composition according to any one of claims 5 to 7 or the pharmaceutical composition according to claim 8 for use in the treatment and/or prevention of rabbit haemorrhagic disease.

14. The fusion protein, VLP, composition or pharmaceutical composition for use according to claim 13, wherein the fusion protein, VLP, composition or pharmaceutical composition is administered prophylactically, and/or the fusion protein, VLP, composition or pharmaceutical composition is administered to a rabbit of the species *Oryctolagus cuniculus.*

## Patentansprüche

1. Fusionsprotein umfassend:
(i) eine N-terminale Arm (NTA)-Domäne umfassend SEQ ID NO: 1 oder eine Sequenz, welche mindestens 98% Sequenzidentität mit SEQ ID NO: 1 hat; und
(ii) eine Hülle (S)-Domäne und eine hervorstehende (P)-Domäne umfassend SEQ ID NO: 2 oder eine Sequenz, welche mindestens 95% Sequenzidentität mit SEQ ID NO: 2 hat.

2. Fusionsprotein nach Anspruch 1, wobei das Fusionsprotein Folgendes umfasst:
(i) SEQ ID NO: 1; und
(ii) SEQ ID NO: 2 oder eine Sequenz, welche mindestens 99% Sequenzidentität mit SEQ ID NO: 2 hat.

3. Fusionsprotein nach einem der Ansprüche 1 oder 2, wobei das Fusionsprotein SEQ ID NO: 3 umfasst.

4. Ein virusähnliches Partikel (VLP) umfassend das Fusionsprotein nach einem der Ansprüche 1 bis 3.

5. Zusammensetzung umfassend das VLP nach Anspruch 4.

6. Zusammensetzung nach Anspruch 5, wobei die Zusammensetzung eine Vielzahl von VLP umfasst und mehr als 50% der VLP in einer Struktur mit T=3 Symmetrie zusammengefügt sind.

7. Zusammensetzung nach Anspruch 5, wobei die Zusammensetzung zusätzlich ein zweites VLP umfasst, welches ein aus RHDVa abgeleitetes VP1-Protein umfasst.

8. Pharmazeutische Zusammensetzung umfassend die Zusammensetzung nach einem der Ansprüche 5 bis 7 und einen pharmazeutisch akzeptablen Träger und/oder ein pharmazeutisch akzeptables Verdünnungsmittel.

9. Nukleinsäure umfassend eine Sequenz, welche das Fusionsprotein nach einem der Ansprüche 1 bis 3 kodiert.

10. Vektor umfassend die Nukleinsäure nach Anspruch 9, wobei der Vektor vorzugsweise aus einer Liste bestehend aus einem Klonierungsvektor, Expressionsvektor, Übertragungsvektor und rekombinanten Virus ausgewählt wird.

11. Bakterienzelle, Hefezelle, Säugetierzelle, Insektenzelle oder Insekt umfassend die Nukleinsäure nach Anspruch 9 und/oder den Vektor nach Anspruch 10.

12. Fusionsprotein nach einem der Ansprüche 1 bis 3, VLP nach Anspruch 4, Zusammensetzung nach einem der Ansprüche 5 bis 7 oder pharmazeutische Zusammensetzung nach Anspruch 8 für deren Verwendung als Medikament.

13. Fusionsprotein nach einem der Ansprüche 1 bis 3, VLP nach Anspruch 4, Zusammensetzung nach einem der Ansprüche 5 bis 7 oder pharmazeutische Zusammensetzung nach Anspruch 8 für deren Verwendung bei der Behandlung und/oder Prävention von hämorrhagischer Kaninchenkrankheit.

14. Fusionsprotein, VLP, Zusammensetzung oder pharmazeutische Zusammensetzung für deren Verwendung nach Anspruch 13, wobei das Fusionsprotein, das VLP, die Zusammensetzung oder die pharmazeutische Zusammensetzung prophylaktisch verabreicht wird, und/oder das Fusionsprotein, das VLP, die Zusammensetzung oder die pharmazeutische Zusammensetzung einem Kaninchen der Art *Oryctolagus cuniculus* verabreicht wird.

## Revendications

1. Protéine de fusion qui comprend :
(i) un domaine de bras N-terminal (NTA) qui comprend SEQ ID NO: 1 ou une séquence qui a au moins 98% d'identité de séquence avec SEQ 10 NO: 1; et
(ii) un domaine d'enveloppe (S) et un domaine de protubérance (P) qui comprennent SEQ ID NO: 2 ou une séquence qui a au moins 95% d'identité de séquence avec SEQ ID NO: 2.

2. Protéine de fusion selon la revendication 1, dans laquelle la protéine de fusion comprend :
(i) SEQ ID NO: 1; et
(ii) SEQ ID NO: 2 ou une séquence qui a au moins 99% d'identité de séquence avec SEQ ID NO: 2.

3. Protéine de fusion selon l'une quelconque des revendications 1 à 2, dans laquelle la protéine de fusion comprend SEQ ID NO: 3.

4. Particule pseudovirale (VLP) qui comprend la protéine de fusion selon l'une quelconque des revendications 1 à 3.

5. Composition qui comprend la VLP selon la revendication 4.

6. Composition selon la revendication 5, dans laquelle la composition comprend une pluralité de VLP et plus de 50% des VLP sont rassemblées dans une structure avec symétrie T=3.

7. Composition selon la revendication 5, dans laquelle la composition comprend en plus une seconde VLP qui comprend une protéine VP1 dérivée à partir de RHDVa.

8. Composition pharmaceutique qui comprend la composition selon l'une quelconque des revendications 5 à 7 et un porteur et/ou diluant pharmaceutiquement acceptable.

9. Acide nucléique qui comprend une séquence qui codifie pour la protéine de fusion selon l'une quelconque des revendications 1 à 3.

10. Vecteur qui comprend l'acide nucléique selon la revendication 9, le vecteur est préférablement sélectionné d'une liste qui consiste en un vecteur de clonage, vecteur d'expression, vecteur de transfert et virus recombinant.

11. Cellule bactérienne, cellule de levure, cellule de mammifère, cellule d'insecte ou insecte qui comprend l'acide nucléique selon la revendication 9 et/ou le vecteur selon la revendication 10.

12. Protéine de fusion selon l'une quelconque des revendications 1 à 3, VLP selon la revendication 4, composition selon l'une quelconque des revendications 5 à 7 ou composition pharmaceutique selon la revendication 8, pour son utilisation comme médicament.

13. Protéine de fusion selon l'une quelconque des revendications 1 à 3, VLP selon la revendication 4, composition selon l'une quelconque des revendications 5 à 7 ou composition pharmaceutique selon la revendication 8, pour son utilisation dans le traitement et/ou la prévention de la maladie hémorragique du lapin.

14. Protéine de fusion, VLP, composition ou composition pharmaceutique pour son utilisation selon la revendication 13, dans lesquelles la protéine de fusion, VLP, composition ou composition pharmaceutique est administrée de manière prophylactique, et/ou la protéine de fusion, VLP, composition ou composition pharmaceutique est administrée à un lapin de l'espèce *Oryctolagus cuniculus.*
